# EUROPEAN PATENT APPLICATION

(11) **EP 3 144 677 A1**
(43) Date of publication of application: **22.03.2017**
(21) Application number: 15185399.1
(22) Date of filing: 16.09.2015
(51) Int. Cl.: G01N 33/92, G01N 21/49

(54) **RAPID TEST FOR BIOMARKER LYSOPHOSPHATIDYLCHOLINE USING FTIR**

(71) Applicant: CETICS Healthcare Technologies GmbH, 73728 Esslingen (DE)
(72) Inventor: MASSING, Ulrich, 79106 Freiburg (DE); UNGER, Miriam, 85373 Ismaning (DE)
(74) Representative: Schrell, Andreas

(57) **Abstract**

The present invention relates to a method for determining the concentration of lysophosphatidylcholine in a biological fluid, a method for determining the state or degree of a disease in a subject, a method for determining whether a subject suffers from a disease and a method for monitoring the temporal course of a disease.

## Description

The present invention relates to a method for determining the concentration of lysophosphatidylcholine in a biological fluid, a method for determining the state or degree of a disease in a subject, a method for determining whether a subject suffers from a disease and a method for monitoring the temporal course of a disease.

Various diseases, preferably cancer, can currently be detected with sufficient reliability only by use of costly tests that, in addition, are quite time-consuming. For the detection of cancer, for instance, imaging techniques, such as x-rays and computer tomography scans, as well as endoscopy or tests on blood can be performed. For instance, for diagnosing prostate cancer a PSA (prostate-specific antigen) test is used, wherein the concentration of the biomarker PSA can be determined by a time-consuming immunoassay.

EP 0 644 412 A1 discloses a method for analyzing clinically relevant liquid samples and sample suspensions that comprises the recording of infrared spectra of dried samples of the liquids or suspensions to be examined and their evaluation by use of multi-parameter evaluation procedures. The evaluation procedure assigns or classifies the samples to be analyzed to classes. The evaluation procedure is calibrated with samples belonging to known classes thus adapting the parameters of the evaluation procedure in a manner such that samples of unknown classification can be assigned to classes. In particular, according to EP 0 644 412 A1, infrared spectra are recorded on a dried film of the sample. This spectroscopic method, however, has the inherent drawback that the samples must be dried for the analysis, meaning that the recorded spectra are spectra of non-native, non-homogeneous samples. Hence, additional processing steps for sample preparation are required, said steps being more time-consuming and making automation of the procedure much more difficult, and information that can only be provided by a sample in its native condition is lost. Moreover, this method is characterized by a high classification or assignment error rate.

A MIR (mid infrared) spectrum of a biological fluid, especially blood plasma, includes spectral contributions from different proteins, cholesterol, urea, different phospholipids and other more dilute compounds. Because each individual component contributes a complex set of several absorptions falling within the mid-IR spectral region, it is impossible to find any single absorption band that can serve as a basis to quantify a single component; coincident absorptions from other species would degrade or completely sabotage the effort.

One of the components in a biological fluid, preferably in a blood plasma, is lysophosphatidylcholine, in the following abbreviated by 'LysoPC', which concentration is known to be constant in the blood plasma of healthy people, ranging from 200 to 400 µmol/L. LysoPC plays an important role as biomarker for cancer diseases and various clinical observations have shown that plasma LysoPC levels are often decreased in advanced metastatic cancer patients, especially when weight loss and inflammatory processes occur. Furthermore, it was found that LysoPC also correlates with the degree of heaviness of the cancer disease. LysoPC-level is also decreased during inflammation and even greatly reduced within a sepsis. In the prior art, LysoPC is quantified by thin layer chromatography or HPLC-MS methods; for both techniques complex and time-consuming sample preparation is required. Thus, it is impossible to run each sample separately and only in batches of several samples. Therefore, in the worst case the LysoPC concentration is available only after a few days.

Thus, there is a severe need for improving the LysoPC detection that can be performed in simple fashion without procedures that would be time- and cost-consuming.

Thus, the object of the present invention to be solved is to provide an improved method for determining the concentration of lysophosphatidylcholine in biological fluids, for example of body fluids in organisms. Because of the need for measuring a huge quantity of samples, particularly for clinical diagnosis, it is necessary to provide a method which allows for a high sample throughput with good reproducibility, especially without any time-consuming pretreatment method steps.

This problem has been solved by the subject-matter of the independent claims.

In particular, the object of the present invention has been solved by a, preferably an in vitro, method for determining the concentration of lysophosphatidylcholine in a biological fluid comprising the steps of:
(a) providing an analysis sample of a biological fluid with an unknown concentration of lysophosphatidylcholine and a correlation between (i) known standard concentrations of lysophosphatidylcholine in standard samples and (ii) predicted standard concentrations of lysophosphatidylcholine in the standard samples, wherein the correlation is obtainable by subjecting at least one IR (infrared) spectrum of at least two standard samples with different known concentrations of lysophosphatidylcholine to a multivariate analysis;
(b) recording an IR spectrum of the analysis sample;
(c) subjecting the IR spectrum recorded in step (b) to a multivariate analysis as to obtain a predicted analysis concentration of lysophosphatidylcholine in the analysis sample and
(d) assigning the predicted analysis concentration of lysophosphatidylcholine to one known standard concentration of lysophosphatidylcholine.

It has been surprisingly found that the concentration of LysoPC in a biological fluid can be determined in a very simple and cost-efficient way with IR (infrared) spectroscopy in combination with multivariate analysis. In accordance with the inventive method the analysis sample of a biological fluid with an unknown concentration of LysoPC can simply be subjected to an IR spectrometer which is preferably coupled with a computer program, especially a software, which is adapted to perform a multivariate analysis and assign the predicted analysis concentration of LysoPC to one specific known standard concentration of LysoPC. By the rapid quantification of LysoPC according to the present invention many benefits including early intervention of therapy and reduction in mortality are provided. It has been surprisingly found that the concentration of LysoPC which is a low molecular weight compound and is present in a biological sample in different forms, that is bounded to different molecules, such as proteins, liquids, can be accurately and reliably determined by a method according to the present invention, preferably by the combination of IR spectroscopy and correlation model provided in step (a).

The term 'lysophosphatidylcholine' relates to any 'lysophosphatidylcholine', also called lysolecithin, belonging to the class of chemical compounds which are derived from phosphatidylcholines. They result from partial hydrolysis of phosphatidylcholines, according to which one of the two fatty acid groups has been removed. The general structural formula of lysophosphatidylcholine is preferably as follows:

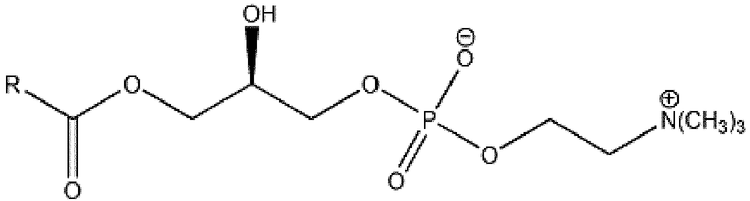

The moiety R represents an arbitrary fatty acid chain, preferably a fatty acid chain comprised naturally in biological fluids of at least one organism.

By the term 'predicted standard or analysis concentration' a concentration is meant which is obtained as a result of subjecting an IR spectrum of a standard or analysis sample with a specific concentration of LysoPC to a multivariate analysis according to the present invention.

The expression 'biological fluid' includes all fluids, preferably liquids, containing biologically relevant substances. In a preferred embodiment of the present invention, a method is provided, wherein the biological fluid is a body fluid of an organism. In a preferred embodiment of the present invention, a method is provided, wherein the biological fluid is selected from the group consisting of blood, blood plasma, blood serum, hemolysate, spinal fluid, urine, salvia, sperm, lymph, synovial fluid, amniotic fluid, lacrimal fluid, cyst fluid, sweat gland secretion and bile.

In context with the present invention, the term 'organism' refers to individual cells, such as prokaryotic and eukaryotic cells, as well as multi-cell organisms, particularly plants, animals and humans. Preferred organisms are selected from the group consisting of *Bos taurus*, *Gallus gallus, Maleagris gallopavo, Mus musculus*, *Ovis ammon, Ratus norwegicus, Sus scrofa* (in general: sheep, chicken, hog) and *Homo sapiens.*

In a preferred embodiment of the present invention, the object of the present invention has been solved by a method, wherein prior to step (a), the following method steps are performed:
(aa) providing at least two standard samples with different known concentrations of lysophosphatidylcholine,
(bb) recording an IR spectrum of the least two standard samples,
(cc) subjecting the IR spectra recorded in step (bb) to a multivariate analysis as to obtain a correlation between (i) known standard concentrations of lysophosphatidylcholine in standard samples and (ii) predicted standard concentrations of lysophosphatidylcholine in the standard samples.

Accordingly, at least two, preferably at least 3, preferably at least 20, preferably at least 100, standard samples with different, but known concentrations of LysoPC are provided in step (aa), which preferably range from 0 to 1000 µmol/L, preferably from 10 to 800 µmol/L, preferably from 20 to 400 µmol/L.

In the subsequent method step (bb) at least one, preferably at least two, preferably at least three, IR (infrared) spectrum/spectra of each standard sample provided in step (aa) is/are recorded as to obtain preferably raw IR spectra. In a preferred embodiment of the present invention, the raw IR spectra recorded in step (b) and/or (bb) are preferably not subjected to any signal processing, especially to an operation selected from the group consisting of baseline correction, normalization, alignment, filtering and cropping. Preferably, the raw IR spectra of the standard samples are obtained by subtracting the noise spectrum of the IR spectrometer from absorption or transmission bands recorded. Preferably, the raw IR spectra of the standard and/or analysis samples are obtained by subtracting the IR water spectrum with the same parameters and devices as the standard and/or analysis samples from the IR spectra of the standard and/or analysis samples recorded with the same IR spectrometer parameters and devices, or by dividing the recorded IR spectra of the standard and/or analysis samples, especially the absorption or transmission bands thereof, by the IR water spectrum, especially the absorption or transmission bands thereof, recorded with the same IR spectrometer parameters and devices.

In the subsequent method step (cc) the IR spectra recorded in step (bb) are subjected to a multivariate analysis as to obtain a correlation between (i) the known standard concentration of LysoPC in said standard samples and (ii) the predicted standard concentration of LysoPC in said standard samples. The known standard concentrations of LysoPC are preferably determined by another method well-known in the state of the art for determining the concentration of biomarkers, especially components present in a biological fluid, preferably by thin layer chromatography or HPLC-MS methods (High Pressure Liquid Chromatography, Mass Spectrometry). Alternatively, the known concentration of LysoPC in standard samples can also be determined by providing aqueous solutions of solely LysoPC, wherein a specific known weight of LysoPC has been added to a specific amount of water.

Preferably, by subjecting the IR spectra recorded in step (bb) to a multivariate analysis, preferably to a PLS regression analysis, in step (cc) one predicted standard concentration value of LysoPC in one standard sample is assigned to one specific known standard concentration of LysoPC as to obtain preferably a pair of values of one predicted standard concentration and one known standard concentration. Preferably, based on said pairs of values a multivariate analysis model is calculated. Preferably, a graph, especially a straight line with a specific gradient, also called slope, is calculated, which have a good linear fit to the data previously obtained, preferably to the pairs of values, and preferably have a R²-value of a least 0.98, preferably 0.99.

In a preferred embodiment of the present invention, a method is provided, wherein the analysis or standard sample is homogenized and/or freed of particulate constituents prior to providing of step (a) and/or step (aa). The body fluid blood is preferably freed of particulate constituents as to provide a serum. The removal of particulate constituents is preferably performed by a centrifugation and/or filtration or ultrafiltration through a membrane of appropriate pore size. The samples provided in step (a) and/or (aa) are preferably native samples.

In a preferred embodiment of the present invention, the standard and analysis samples provided in step (a) or (aa) are subjected, preferably prior thereto, to filtration, particularly ultrafiltration, centrifugation and/or dialysis. By said treatment it is preferably possible to obtain a body fluid fraction that is optimized, namely that provide the largest possible specific spectral change. In this manner, it is possible to reduce appreciably the total, highly complex spectral information thus offering the possibility of obtaining information about the clinical picture with the aid of data analysis.

In a preferred embodiment of the present invention, the standard and analysis samples provided in step (a) or (aa) are subjected, preferably prior thereto, to an electrochemically induced difference analysis, wherein electrodes are integrated with the measuring cell. In this manner, with the aid of an applied potential, certain constituents, particularly biomolecules such as proteins (for example heme proteins), can be selectively oxidized or reduced depending on their midpoint potential. The changes induced in this manner, preferably changes of an electronic or structural kind, can be used as recognition features. By varying the applied potential, it is thus possible to selectively modify various constituents. Thus, by electrochemically induced difference analysis based on the selection of only redox-active components of the body fluid and by appropriate selection of the potential range, it is possible to achieve an advantageous reduction of the complex spectral information and the detection sensitivity being increased.

Accordingly, in a preferred embodiment of the present invention, a method is provided, wherein the measuring cell contains integrated electrodes for the reduction and/or oxidation of biomolecules in the sample. The electrodes are preferably integrated in the measuring cell in the form of micro structured networks, to permit the electrochemically induced difference analysis (difference spectroscopy). Such measuring cells are described, for example, by Moss, A. D., Nabedryk, E., Breton, J. and Mäntele, W. (1990) Eur. J. Biochem. 187, 565-572.

The method steps carried out prior to the provision of the samples and/or recording of the IR spectrum do not preferably change the 'native' character of the samples. Hence, the analysis sample provided for and/or subjected to the IR spectroscopy is characterized in that the constituents contained therein are present under conditions, particularly as regards the water content, salt content and/or pH, and optionally temperature, which are the same as those of the body fluid in the organism from which the sample was taken. This means that the biomolecules (protein, nucleic acids etc.) contained in the body fluid in question are preferably not in a denatured state. This is in contrast to the method described, for example, in EP 0 644 412 A1, for which a fluid sample must be dried on a carrier material before the IR analysis is performed.

Furthermore, the substances dissolved in the body fluid are present in most cases in similar concentrations so that, on the one hand, the native character of the body fluid is retained and, on the other, a direct comparison of the samples is possible. Optionally, it is necessary to standardize the data concerning the path length of the measuring cell used to the path length of the reference measuring cell.

In a preferred embodiment of the present invention, a method is provided, wherein the recording of the IR spectra in step (b) and/or in step (bb) is carried out with the aid of an FTIR spectrometer and/or FTIR microscope.

Suitable apparatus for carrying out the inventive method is preferably an analytical apparatus comprising an FTIR spectrometer, related pumps and a suitable measuring cell, designed for recording FTIR spectra of a sample of a biological fluid.

In a preferred embodiment of the present invention, a method is provided, wherein the IR spectra in step (b) and/or (bb) are recorded with the aid of a measuring cell with a path length of not more than 30 µm, preferably with a path length of from 3 to 12 µm and most preferably not more than 10 µm.

Suitable optical materials for the method of the invention are, in general, all materials that are transparent to IR in the indicated wave number range or partial range, preferably calcium fluoride (CaF₂), barium fluoride (BaF₂) zinc selenide (ZnSe), silicon (Si), germanium (Ge), diamond (C) and thin polymer films. Optionally, the materials can be coated with thin, water-insoluble layers of, for example, Parylene, PTFE or PE. In this manner, special properties can be imparted to the short-path cell. For example, such materials can be used to reduce the interaction between the window material or cell material and the biological sample or to isolate water-soluble window materials from the water-containing sample solution. This provides a larger selection of optical materials and thus a wider spectral range. Therefore, water-soluble potassium bromide (KBr), for example, can also be used as window material. In a preferred embodiment of the method of the invention, the recording of the IR spectra in the aforesaid defined steps (b) and (bb) is carried out by means of a spectrometer designed for Fourier transform infrared spectroscopy (FTIR), which enables fast recording and evaluation of the IR spectra. The IR spectra are preferably recorded in transmission and/or reflection and/or attenuated total reflection (ATR) mode, preferably recorded by the transmission technique. The information content of IR spectra of the biological fluids, preferably body fluids, analyzed according to the invention is particularly high in the middle IR range. For this reason, the recording of the IR spectra in step (b) and/or step (bb) of the method according to the present invention is preferably done at a wave number from 950 to 3050 cm⁻¹, preferably from 1000 to 3000 cm⁻¹, particularly from 1000 to 1275 cm⁻¹ and from 2700 to 3020 cm⁻¹. By using the wave numbers of from 1000 to 1275 cm⁻¹ and from 2700 to 3020 cm⁻¹ an improved and more precise correlation model can be provided.

According to the invention, the IR spectra of the samples recorded in step (bb) for which the concentration of LysoPC in the biological fluid, for example the body fluid of an organism, is known, are subjected in step (cc) to a multivariate analysis. To this end, the values obtained by recording the IR spectrum in question are preferably first digitized. The digitized spectrum is then subjected to the multivariate analysis. Multivariate analyses that can be used according to the invention are preferably regression analysss, preferably including the use of a PLS regression algorithm, that are commercially available as software programs.

In a preferred embodiment of the present invention, a method is provided, wherein the multivariate analysis is a PLS regression analysis. By the usage of a PLS regression analysis it is advantageously possible to assign or convert the predicted analysis concentration to one specific known standard concentration, preferably by dividing the predicted analysis concentration by a, preferably constant, factor, preferably with the gradient of the straight line obtained from the PLS regression analysis in step (cc).

It is preferred to develop the regression model of known and predicted standard concentration of LysoPC on a rather large number, for example more than 100, spectra, because the error rate decreases with the size of the data set available for the multivariate analysis. As stated above, the multivariate analysis of the IR spectra is preferably carried out with the aid of software-controlled data processing. The correlation obtained is preferably stored in an electronic data carrier being then available for the assignment with the corresponding data records of an IR spectrum of an analysis sample.

Preferably, the analysis concentration of LysoPC in the analysis sample is determined by another method for determining the concentration of LysoPC as to verify the predicted analysis concentration of LysoPC. Preferably, the predicted analysis concentration of LysoPC obtained in step (c) and the verified concentration of LysoPC in the analysis sample are used in step (cc) as further predicted and known standard concentration of LysoPC, as to especially increase the reproducibility and reliability of the method according to the present invention.

The three following embodiments of one-piece short-path cells for fluids are preferably used for the analytical methods:

### Type 1: One-piece micro structured flow-through cell

Such cells are commercially available and have the following characteristics: High pressure resistance, preferably for example 10-100 bar. This is advantageous in case of high flow resistance during the filling of flow-through cells, particularly those with path lengths ranging from 1 to 15 µm;
Small filling volume, preferably 0.05 to 3µL; hence only very small sample quantities are suitable;
Automated high throughput is possible; the filling and rinsing of the cells can be accomplished very quickly;
Fast pressure relaxation, preferably of <10 ms; needed for high sample throughput;
Such cells retain a constant path length during the sample analysis despite the varying pressure conditions. The deviation remains below the detection limit of the IR analysis and, hence, does not produce interfering signals. The reproducibility of the method of the invention is thus extremely improved;

Integration with micro structured electrodes is possible.

By the integration of electrodes with the IR measuring cell, preferably of microstructured electrodes with the one-piece microflow-through cell, it is possible to impart to the method of the invention the following additional advantages:
Complexity reduction for samples with redox-active components in the body fluid can be achieved. Data analysis based on the total spectrum and the detailed (high-resolution) electrochemically induced difference spectrum is possible providing new (additional) spectral information.

By integration of the cell with microstructured electrodes, scannable potential intervals can be evaluated for spectral changes specific for a potential interval.

It is possible to evaluate a potential range with maximum disease-specific spectral change optimized for a specific disease.

### Type 2: One-piece microstructured diffusion mixing cell (also called thin layer sensor)

Such measuring cells are known from the prior art and have the following properties:
No cleaning is necessary, because the cells are discarded after use (single-use cells);
Constant path length;

The sample volume needed is small, preferably <1 µL, in particular 50-200 nL;

Fast pressure relaxation and high pressure resistance are not needed, because: single-use, disposable cells or array are used (no time-consuming rinsing and cleaning is necessary; handling is simple when working with pathogenic biomaterial (for example with samples assigned to safety class S2); filling occurs by capillary force; Suitable for 'point-of-care' use.

### Type 3: one-piece capillary force short-path cell

A measuring cell of this type is described in DE 197 39 126 and has the following properties:
Small sample volume, preferably <5 µL;
Fast relaxation and high pressure resistance are not needed, because the cell is of the single-use, disposable type; filling occurs by capillary force;
Integration with micro structured electrodes is possible.

According to a preferred embodiment of the method of the invention, the recording, subjecting and assigning steps are fully automated for a high throughput of several thousand, preferably 1 to 3000, preferably 50 to 2000, preferably 100 to 1000, preferably at least 5000, recordings and evaluations per day per instrument. To this end, the method of the invention can be carried out with the aid of a measuring apparatus in which the individual components such as pumps, sampling loops, control valves, mixers etc., are designed for operation with very small volumes and high pressures, such as those encountered, for example, in high pressure liquid chromatography (HPLC). The control of these individual components is preferably accomplished by computer. The use of HPLC components in conjunction with a micro flow-through cell having a path length of 1 to 15 µm requires very small samples of preferably <20 µL. More preferable are flowthrough systems requiring samples from 1 to 10 µL and particularly 5 µL. Preferably, the volume of the sample, preferably of the standard and/or the analysis sample, is 1 to 100 µL, preferably 5 to 90 µL, preferably 40 to 80 µL, preferably depending on the application and the adjustment of measuring parameters.

Preferably, by the usage of said small volumes of the standard and/or the analysis sample and/or the herein defined path-length of the measuring cells the measuring in biological samples, i.e. water based samples, can be performed.

The spectra resolution of the spectrometer is preferably between 2 and 16 cm⁻¹, preferably 4 cm⁻¹.

In carrying out the inventive method, particularly when using HPLC components, high pressures from 1 to 100 bar are generated depending on the speed at which the measuring cell is filled. To ensure the reproducibility of the method of the invention and thus the reliable determination of the concentration of LysoPC in the biological fluid being examined, the measuring cell during the recording of the spectrum shows a path length deviation of preferably less than 1 nm from the native path length despite the varying high pressure load during filling and rinsing of the cell.

In particular, the method of the invention is carried out, for example, in an aforesaid flow-through apparatus, as follows: With the pump running, the samples, for example, blood serum, blood plasma etc., are fed via a sampling loop valve, to a transport medium, preferably water, or an aqueous buffer solution and then transported into the FTIR measuring cell. Once the sample is in the measuring cell, the flow is stopped and the FTIR spectrum of the sample is recorded. The cell is then rinsed with transport medium. By use of sampling loop valves, the sampling loop can be refilled even while recording is being carried out. In this manner, the sample throughput is limited almost exclusively by the duration of the recording time, in the case of FTIR recording 1 to 5 min, preferably 2 to 3 minutes, as a result of which the system is particularly well suited for automation for a large number of samples.

The above system is suitable for manual, semiautomatic as well as fully automatic execution of the method of the invention. In the case of automated, high sample-throughput, the above recording system is preferably computer-controlled and fitted with HPLC-compatible components. In such a system, the sample can be fed, for example, from standardized microtiter plates.

In an automated version of the method of the invention, the recording of the IR spectra is preferably done with the aid of the aforesaid one-piece, microstructured flow-through cell into which can optionally be integrated electrodes for carrying out an electrochemically induced difference analysis of the biological fluid.

In contrast to the tests thus far performed on dead organisms, the method of the invention can be performed with living organisms and, moreover, does not involve significant procedures. The method of the invention involving up to or more 5000 determinations per day per apparatus can be carried out very quickly. By avoiding high labor costs and large expenses, considerable cost reduction can be achieved for the individual analyses.

Moreover, only very small amounts of sample, preferably <20 *µL,* are needed, particularly when HPLC components are used.

Moreover, the standard sample used for providing the correlation between predicted and known standard concentration of LysoPC should preferably show the same degree of dehydration, otherwise different band shifts would hamper an unambiguous assignment of the predicted analysis concentration to one specific known standard concentration of LysoPC.

Also for better reproducibility, the recording of spectra of the analysis samples is performed by the same recording technique and parameters as for the standard samples.

The object of the present invention has also been solved by a method for determining the state or degree of a disease in a subject comprising the steps of:
(a) providing an analysis sample of a biological fluid with an unknown concentration of lysophosphatidylcholine and
   a correlation between (i) known standard concentrations of lysophosphatidylcholine in standard samples and (ii) predicted standard concentrations of lysophosphatidylcholine in the standard samples, wherein the correlation is obtainable by subjecting at least one IR spectrum of at least two standard samples with different known concentrations of lysophosphatidylcholine to a multivariate analysis;
(b) recording an IR spectrum of the analysis sample;
(c) subjecting the IR spectrum recorded in step (b) to a multivariate analysis as to obtain a predicted analysis concentration of lysophosphatidylcholine in the analysis sample and
(d) assigning the predicted analysis concentration of lysophosphatidylcholine to one known standard concentration of lysophosphatidylcholine.

The object of the present invention has also been solved by a method for determining whether a subject suffers from a disease comprising the steps of:
(a) providing an analysis sample of a biological fluid with an unknown concentration of lysophosphatidylcholine which is obtained from said subject and
   a correlation between (i) known standard concentrations of lysophosphatidylcholine in standard samples and (ii) predicted standard concentrations of lysophosphatidylcholine in the standard samples, wherein the correlation is obtainable by subjecting at least one IR spectrum of at least two standard samples with different known concentrations of lysophosphatidylcholine to a multivariate analysis;
(b) recording an IR spectrum of the analysis sample;
(c) subjecting the IR spectrum recorded in step (b) to a multivariate analysis as to obtain a predicted analysis concentration of lysophosphatidylcholine in the analysis sample and
(d) assigning the predicted analysis concentration of lysophosphatidylcholine to one known standard concentration of lysophosphatidylcholine.

In a preferred embodiment of the present invention, a method is provided, wherein it is determined that a subject suffers from a disease if the analysis concentration of LysoPC in the analysis samples deviates from a pre-determined value, preferably range of concentration of LysoPC. The pre-determined value of concentration of LysoPC is selected from 200 µM/L, preferably 180 µM/L, preferably 150 µM/L. The pre-determined range of concentration of LysoPC is preferably 200 to 400 µM, preferably 250 to 350 µM, preferably 280 to 320 µM.

The methods according to the present invention are preferably methods for determining the state or degree of a disease, preferably cancer in a subject or whether a subject has a disease, preferably cancer, based solely on an analytical procedure and said determination is provided by an automatic system comprising an IR spectrometer and a device for carrying out a multivariate analysis. Said methods do preferably not comprise a step of diagnosis for curative purposes stricto sensu representing the deductive medical or veterinary decision phase as a purely intellectual exercise. Preferably, the method for determining whether a subject suffers from a disease, preferably cancer or the state or degree of a disease, preferably cancer in a subject is not a diagnostic method. Thus, preferably diagnostic methods are excluded from the scope of said method claims.

The object of the present invention has also been solved by a method for a diagnosis, preferably differential diagnosis, comprising the steps of:
(a) providing an analysis sample of a biological fluid with an unknown concentration of lysophosphatidylcholine and
   a correlation between (i) known standard concentrations of lysophosphatidylcholine in standard samples and (ii) predicted standard concentrations of lysophosphatidylcholine in the standard samples, wherein the correlation is obtainable by subjecting at least one IR spectrum of at least two standard samples with different known concentrations of lysophosphatidylcholine to a multivariate analysis;
(b) recording an IR spectrum of the analysis sample;
(c) subjecting the IR spectrum recorded in step (b) to a multivariate analysis as to obtain a predicted analysis concentration of lysophosphatidylcholine in the analysis sample,
(d) assigning the predicted analysis concentration of lysophosphatidylcholine to one known standard concentration of lysophosphatidylcholine, and
(e) using the assigned standard concentration of lysophosphatidylcholine for determining whether a subject suffers from cancer and/or the state or degree of the cancer.

In this manner, by the method of the invention, an analysis sample of a body fluid of unknown concentration of LysoPC is measured, subjected to the multivariate analysis and, by comparison of the predicted analysis concentrations of LysoPC the IR spectrum of the analysis sample subjected to the multivariate analysis with the previously obtained and stored correlation of the IR spectra of the known standard samples, is assigned to a condition, for example condition A or condition B. Preferably, the condition A means 'ill' or 'with findings' or 'cancerous'. Preferably, the condition B means 'not ill' or 'without findings' or 'not cancerous'.

Preferably, the concentration of LysoPC in a biological fluid, especially in a body fluid, is used a marker for the severity, state or grade of a disease, especially of cancer. The lower the concentration of LysoPC in a biological fluid, especially a body fluid, is, the higher the severity, state or grade of the disease, especially of cancer, is.

The concentration of lysoPC in a biological fluid, preferably of an organism, preferably of a human being, suffering from a disease, can be higher or lower than the lysoPC level in a biological fluid of a subject, preferably of an organism, preferably of a human being, not suffering from said disease.

In a preferred embodiment of the present invention, the disease is preferably a disease in which the lysoPC level in a biological fluid of a subject, preferably of an organism, preferably of a human being, suffering from said disease is elevated or a disease in which the lysoPC level in a biological fluid of a subject, preferably of an organism, preferably of human being, suffering from said disease is reduced compared to the lysoPC level in a biological fluid from a subject, preferably of an organism, preferably of a human being, not suffering from said disease.

Preferably, the disease or pathological condition is selected from the group consisting of cancer, leukemia, renal cell carcinoma, colon cancer, prostate cancer, ovarian carcinoma, multiple myeloma, obesity, type II diabetes, sepsis, pneumonia, Alzheimer's disease and cardiovascular diseases.

The cardiovascular disease is preferably selected from the group consisting of ischemic heart disease, stroke, hypertensive heart disease, rheumatic heart disease, aortic aneurysms, cardiomyopathy, arterial fibrillation, congenital heart disease, endocarditis and peripheral artery disease.

The cancer is preferably selected from leukemia, renal cell carcinoma, colon cancer, prostate cancer, ovarian carcinoma, and multiple myeloma.

The lysoPC level is elevated in subjects suffering ovarian carcinoma and multiple myeloma. The lysoPC level is reduced in subjects suffering leukemia, renal cell carcinoma, colon cancer, obesity, type II diabetes, sepsis, pneumonia, Alzheimer's disease and cardiovascular diseases.

Thus, the method of the invention can preferably be used to detect pathological conditions, preferably cancer, which means that the sample of a body fluid of an organism is assigned to the condition 'ill' or 'with findings' or 'cancerous' or to the condition 'not ill' or 'without findings' or 'not cancerous'.

In a preferred embodiment, the methods according to the present invention are methods aiding in the diagnosis of a disease, comprising the steps (a) to (d), preferably in combination with steps (aa) to (cc). In said methods the concentration of lysophosphatidylcholine is preferably determined in accordance with the present invention as to verify or nullify the presence of a disease.

The term 'disease' in accordance with the present invention is understood as a disease wherein the lysoPC concentration level of a biological fluid of a subject, especially of a human being, deviates from lysoPC concentration level of a healthy subject, especially of a human being. The lysoPC concentration level of a healthy subject, preferably of a healthy human being, is preferably 200 to 400 µM, preferably 250 to 350 µM, preferably 280 to 320 µM. Any disease where the lysoPC concentration level usually deviates from the standard lysoPC level falls under the definition of 'disease' in accordance with the present invention. Thus, even if some subjects suffering from said disease, but have no altered or deviated lysoPC level in their biological fluid, said disease is understood as a disease in accordance with the present invention as long as preferably at least 90%, preferably at least 95%, preferably at least 99%, of all subjects suffering from said disease have an altered or deviated lysoPC level, that is a lysoPC level higher or lower than 200 to 400 µM, preferably 250 to 350 µM, preferably 280 to 320 µM.

The present invention also relates to a method for monitoring the temporal course of a disease, preferably the temporal change in its state or degree, wherein the method comprises the following steps:
(i) determining the concentration of lysophosphatidylcholine in a biological fluid at a first time point and
(ii) determining the concentration of lysophosphatidylcholine in a biological fluid at a second time point,
wherein the determination of the concentration of lysophosphatidylcholine in step (i) and/or (ii) is determined by
(a) providing an analysis sample of a biological fluid with an unknown concentration of lysophosphatidylcholine and a correlation between (i) known standard concentrations of lysophosphatidylcholine in standard samples and (ii) predicted standard concentrations of lysophosphatidylcholine in the standard samples, wherein the correlation is obtainable by subjecting at least one IR spectrum of at least two standard samples with different known concentrations of lysophosphatidylcholine to a multivariate analysis;
(b) recording an IR spectrum of the analysis sample;
(c) subjecting the IR spectrum recorded in step (b) to a multivariate analysis as to obtain a predicted analysis concentration of lysophosphatidylcholine in the analysis sample and
(d) assigning the predicted analysis concentration of lysophosphatidylcholine to one known standard concentration of lysophosphatidylcholine.

Preferably, the first time point is - seen from a temporal perspective - different from the second time point.

Preferably, the method for monitoring the temporal course of a disease further comprises the steps of
(aa) providing at least two standard samples with different known concentrations of lysophosphatidylcholine,
(bb) recording at least one IR spectrum of the least two standard samples,
(cc) subjecting the IR spectra recorded in step (bb) to a multivariate analysis as to obtain a correlation between (i) known standard concentrations of lysophosphatidylcholine in standard samples and (ii) predicted standard concentrations of lysophosphatidylcholine in the standard samples.

Preferably, the biological fluid, preferably the body fluid, in which the concentration of lysoPC is determined in step a) is a biological fluid, preferably a body fluid, of a subject, preferably of a human being suffering from a disease in accordance with the present invention.

Alternatively or additionally, the biological fluid, preferably the body fluid, in which the concentration of lysoPC is determined in step b) is preferably a biological fluid, preferably a body fluid, of a subject, preferably of a human being suffering from a disease in accordance with the present invention.

Alternatively, the biological fluid, preferably the body fluid, in which the concentration of lysoPC is determined in step b) is preferably a biological fluid, preferably a body fluid, of a subject, preferably of a human being which suffered from a disease in accordance with the present invention. Preferably, said body fluid is a body fluid from a subject, preferably of a human being which is treated or was treated against the disease.

In a preferred embodiment of the present invention, the concentration of lysoPC is determined at two different, preferably at least three different, preferably at least five different, preferably at least ten different, time points. Preferably, at least one, preferably at least two, preferably at least five, preferably at least ten, preferably all determinations of the concentration of lysoPC is performed by the method according to the present invention comprising the steps (a) to (d).

Said method allows for a very simple and accurate determination of the lysoPC concentration in a biological fluid, preferably a body fluid of a subject, and can thereof be used for monitoring the temporal course, preferably the temporal change of the state or degree of a disease depending upon the deviation of lysoPC levels in a biological fluid in an easy and hardly time-consuming manner. By said specific method it can easily be determined whether the treatment against a disease is/was successful and said method can be used in follow-up examinations for aiding the state of health in a hardly time and cost-consuming manner. Accordingly, the determination of the concentration of lysoPC in a biological fluid of a subject suffering or suffered from a disease, wherein the lysoPC level in a biological fluid is indicative for said disease, can be used as parameter for aiding in the diagnosis of said disease, its course and its recurrence. By determining the concentration of lysoPC in accordance with the present invention, the probability for resulting in the right diagnosis will be further elevated and the need and extent of the therapy against a specific disease can be easily evaluated.

In addition to the concentration of lysoPC a concentration of at least one further biomarker is preferably determined by the methods according to the present invention.

The object of the present invention is preferably solved by the subject-matter of the dependent claims.

Furthermore, the present invention is illustrated by the following examples.

### 1. Sample Preparation

For this study, blood plasma samples of prostatic-positive cancer patients were shipped on dry ice (transport time of samples was one day). After arrival the samples were stored in an -80 °C ultra-low freezer until measurements were performed.

For the IR-spectroscopic investigations all samples were defrosted at 4 °C and then stirred with a Vortex device. In the next step, the plasma samples were centrifuged (4 °C, 3000 rcf/g, 5 min) and aliquoted into a 500 µL reaction tubes tube.

### 2. FT-IR spectroscopy

The IR spectra are recorded by a specially constructed FT-IR spectrometer based on the AquaSpec FT-MIR (Fourier Transform mid-infrared spectroscopy) technique that measures mid-infrared spectra of liquid biological samples (here blood plasma) in transmission mode. The centerpiece is a patent-registered flow-through transmission cell, consisting of a path length of approx. 9 µm and therefore optimized for the measurement of aqueous solutions. Mid-infrared spectra are measured in the range of 3050-950 cm⁻¹ with a spectral resolution of 4 cm⁻¹. MIR spectra are normalized and standardized by build-in algorithms. The samples are recorded in their native state without further sample preparation. The sampling is carried out from 500 µL reaction tubes (e.g. Eppendorf) using an automated robot system with a direct injection into the flow through transmission cell.

### 3. Results and Discussion

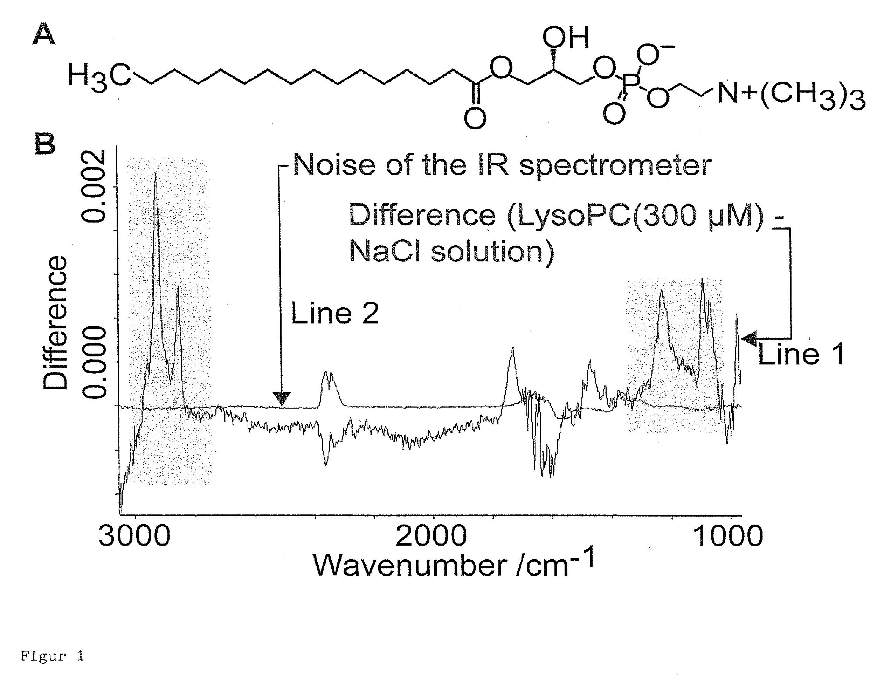

The distinctive IR absorption bands belonging to LysoPC are apparent in the IR difference spectrum used to build the PLS regression model (see Figure 1). Moreover, the specific absorption bands of LysoPC are assigned to their vibrational mode and compared to the chemical structure of LysoPC as depicted in Figure 1A and 1B. Additionally, IR-specific spectral features of LysoPC are highlighted in Figure 1B.

Figure 1B shows the difference spectra of LysoPC (line 1) and the calculated noise of the IR spectrometer based on the difference of two repeated measurements (line 2).

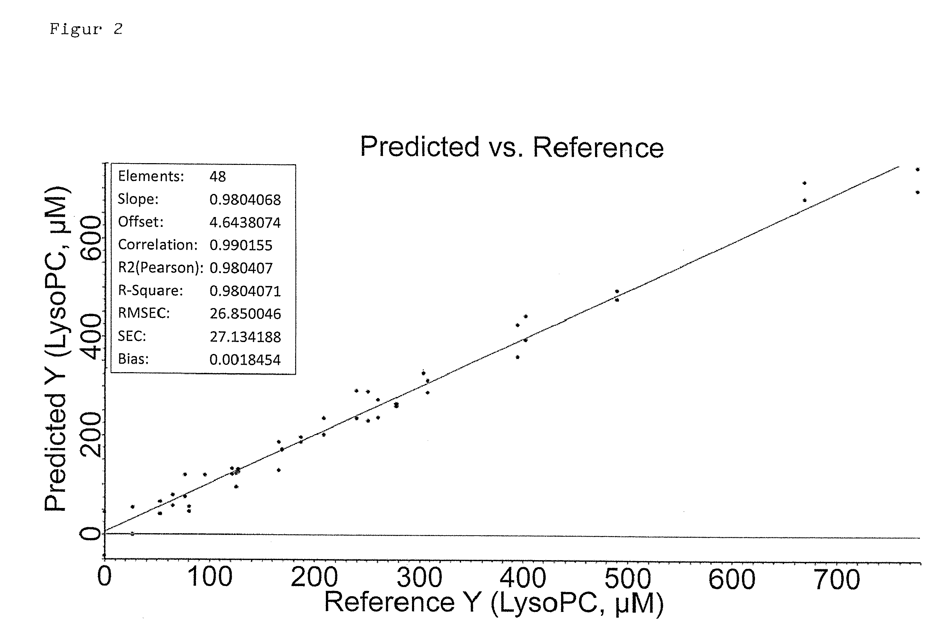

The important IR absorptions for LysoPC are within the wavenumber range of 3015-2735 cm⁻¹ and 1273-1005 cm⁻¹. Accordingly, the PLS regression was performed on the same range. The PLS regression model gives a good linear fit to the data (R²=0.9805, RMSEC=26.85 µM/L), as shown in Figure 2.

Figure 2 shows a PLS regression model of LysoPC. The model was developed in the wavenumber regions 3015-2735 cm⁻¹ and 1273-1005 cm⁻¹.

### 4. Conclusion

This study demonstrates that our proposed IR spectroscopic approach in combination with chemometric applications has the potential to become a clinical method for quick determination of LysoPC in blood plasma. In general our FT-IR spectrometer is a simple, rapid and cost-effective solution for the quantification of parameters in any biological liquid sample.

## Claims

1. Method for determining the concentration of lysophosphatidylcholine in a biological fluid comprising the steps of:
(a) providing
an analysis sample of a biological fluid with an unknown concentration of lysophosphatidylcholine and
a correlation between (i) known standard concentrations of lysophosphatidylcholine in standard samples and (ii) predicted standard concentrations of lysophosphatidylcholine in the standard samples, wherein the correlation is obtainable by subjecting at least one IR spectrum of at least two standard samples with different known concentrations of lysophosphatidylcholine to a multivariate analysis;
(b) recording an IR spectrum of the analysis sample;
(c) subjecting the IR spectrum recorded in step (b) to a multivariate analysis as to obtain a predicted analysis concentration of lysophosphatidylcholine in the analysis sample and
(d) assigning the predicted analysis concentration of lysophosphatidylcholine to one known standard concentration of lysophosphatidylcholine.

2. Method according to claim 1, wherein prior to step (a), the following method steps are performed:
(aa) providing at least two standard samples with different known concentrations of lysophosphatidylcholine,
(bb) recording at least one IR spectrum of the at least two standard samples,
(cc) subjecting the IR spectra recorded in step (bb) to a multivariate analysis as to obtain a correlation between (i) known standard concentrations of lysophosphatidylcholine in standard samples and (ii) predicted standard concentrations of lysophosphatidylcholine in the standard samples.

3. Method according to one of claims 1 and 2, wherein the sample(s) are homogenized and/or freed of particulate constituents prior to providing of step (a) and/or step (aa).

4. Method according to one of the preceding claims, wherein the recording of the IR spectra in step (b) and/or in step (bb) is carried out at a wave number from 950 to 3050 cm⁻¹.

5. Method according to one of the preceding claims, wherein the recording of the IR spectra in step (b) and/or in step (bb) is carried out with the aid of an FTIR spectrometer and/or FTIR microscope.

6. Method according to any one of the preceding claims, wherein the IR spectra in step (b) and/or (bb) are recorded with the aid of a measuring cell with a path length of not more than 30 µ*m*.

7. Method according to one of the preceding claims, wherein the recording of the IR spectra in step (b) and/or step (bb) is carried out with the aid of a measuring cell with a path length from 3 to 12 *µm*.

8. Method according to one of the preceding claims, wherein the measuring cell contains integrated electrodes for the reduction and/or oxidation of biomolecules in the sample.

9. Method according to one of the preceding claims, wherein the multivariate analysis is a PLS regression analysis.

10. Method according to one of the preceding claims, wherein the biological fluid is a body fluid of an organism.

11. Method according to one of the preceding claims, wherein the biological fluid is selected from the group consisting of blood, blood plasma, blood serum, hemolysate, spinal fluid, urine, saliva, sperm, lymph, synovial fluid, amniotic fluid, lacrimal fluid, cyst fluid, sweat gland secretion, bile and mixtures thereof.

12. Method according to claim 10 or 11, wherein the organism is selected from the group consisting of *Bos taurus*, *Gallus gallus, Maleagris gallopavo, Mus musculus*, *Ovis ammon, Rattus norwegicus, Sus scrofa* and *Homo sapiens.*

13. Method for determining the state or degree of disease in a subject, comprising the steps of:
(a) providing
an analysis sample of a biological fluid with an unknown concentration of lysophosphatidylcholine and
a correlation between (i) known standard concentrations of lysophosphatidylcholine in standard samples and (ii) predicted standard concentrations of lysophosphatidylcholine in the standard samples, wherein the correlation is obtainable by subjecting at least one IR spectrum of at least two standard samples with different known concentrations of lysophosphatidylcholine to a multivariate analysis;
(b) recording an IR spectrum of the analysis sample;
(c) subjecting the IR spectrum recorded in step (b) to a multivariate analysis as to obtain a predicted analysis concentration of lysophosphatidylcholine in the analysis sample and
(d) assigning the predicted analysis concentration of lysophosphatidylcholine to one known standard concentration of lysophosphatidylcholine.

14. Method for determining whether a subject suffers from a disease, comprising the steps of:
(a) providing
an analysis sample of a biological fluid with an unknown concentration of lysophosphatidylcholine which is obtained from said subject and
a correlation between (i) known standard concentrations of lysophosphatidylcholine in standard samples and (ii) predicted standard concentrations of lysophosphatidylcholine in the standard samples, wherein the correlation is obtainable by subjecting at least one IR spectrum of at least two standard samples with different known concentrations of lysophosphatidylcholine to a multivariate analysis;
(b) recording an IR spectrum of the analysis sample;
(c) subjecting the IR spectrum recorded in step (b) to a multivariate analysis as to obtain a predicted analysis concentration of lysophosphatidylcholine in the analysis sample and
(d) assigning the predicted analysis concentration of lysophosphatidylcholine to one known standard concentration of lysophosphatidylcholine.

15. Method according to claim 14, wherein it is determined that a subject suffers from the disease, if the analysis concentration of lysophosphatidylcholine in the analysis sample deviates from a predetermined value, preferably range, of concentration of lysophosphatidylcholine.

16. Method according to claims 14 and 15, wherein the disease is selected from the group consisting of cancer, leukemia, renal cell carcinoma, colon cancer, prostate cancer, ovarian carcinoma, multiple myeloma, obesity, type II diabetes, sepsis, pneumonia, Alzheimer's disease and cardiovascular diseases.

17. A method for monitoring the temporal course of a disease, wherein the method comprises the following steps:
(i) determining the concentration of lysophosphatidylcholine in a biological fluid at a first time point and
(ii) determining the concentration of lysophosphatidylcholine in a biological fluid at a second time point,
wherein the determination of the concentration of lysophosphatidylcholine in step (i) and/or (ii) is determined by
(a) providing an analysis sample of a biological fluid with an unknown concentration of lysophosphatidylcholine and a correlation between (i) known standard concentrations of lysophosphatidylcholine in standard samples and (ii) predicted standard concentrations of lysophosphatidylcholine in the standard samples, wherein the correlation is obtainable by subjecting at least one IR spectrum of at least two standard samples with different known concentrations of lysophosphatidylcholine to a multivariate analysis;
(b) recording an IR spectrum of the analysis sample;
(c) subjecting the IR spectrum recorded in step (b) to a multivariate analysis as to obtain a predicted analysis concentration of lysophosphatidylcholine in the analysis sample and
(d) assigning the predicted analysis concentration of lysophosphatidylcholine to one known standard concentration of lysophosphatidylcholine.
